Europäisches Patentamt
European Patent Office
Office européen des brevets

(11) Publication number: 0 280 521 A2

# EUROPEAN PATENT APPLICATION

(21) Application number: 88301567.9

(22) Date of filing: 24.02.88

(51) Int. Cl.4: C 07 D 501/22
A 61 K 31/545

(30) Priority: 27.02.87 GB 8704621

(43) Date of publication of application: 31.08.88 Bulletin 88/35

(84) Designated Contracting States: AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Newall, Christopher Earle
33 Elm Grove Road
London W5 (GB)

Looker, Brian Edgar
28 Middleton Road
Greenford Middlesex (GB)

Weir, Niall Galbraith
62 Rydal Gardens
Wembley Middlesex (GB)

Weingarten, Gordon Gad
35 Princes Park Avenue
London NW11 (GB)

(74) Representative: Holmes, Michael John et al
Frank B. Dehn & Co. European Patent Attorneys Imperial House 15-19 Kingsway
London, WC2B 6UZ, (GB)

The title of the invention has been amended (Guidelines for Examination in the EPO, A-III, 7.3).

(54) **Cephalosporin compounds, process for their preparation and pharmaceutical compositions containing them.**

(57) Compounds of formula (I)

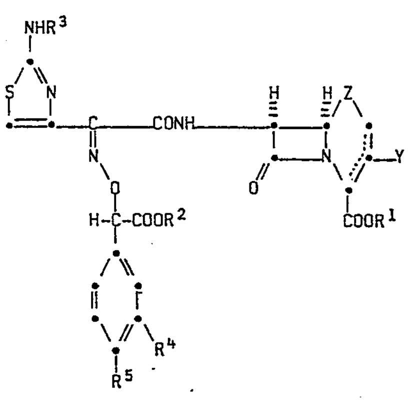

(I)

wherein
$R^1$ and $R^2$, which may be the same or different, each represents a hydrogen atom or a carboxyl blocking group;
$R^3$ represents a hydrogen atom or an amino protecting group;
$R^4$ and $R^5$ each represents a hydroxy or substituted hydroxy group or $R^4$ and $R^5$ together represent a cyclic protected diol group;
Y represents a methyl or vinyl group;
Z is -S- or -SO- (α- or β-) and the dotted line bridging the 2-, 3-, and 4-positions indicates that the compound is a ceph-2-em or ceph-3-em compound;
and salts and solvates thereof.

EP 0 280 521 A2

**Description**

CHEMICAL COMPOUNDS

This invention relates to improvements in or relating to cephalosporins. More particularly it relates to new cephalosporin compounds and derivatives thereof having valuable antibiotic activity.

The cephalosporin compounds in this specification are named with reference to 'cepham' after J. Amer. Chem. Soc., 1962, 84, 3400, the term 'cephem' referring to the basic cepham structure with one double bond.

Cephalosporin antibiotics are widely used in the treatment of diseases caused by pathogenic bacteria in human beings and animals, and are especially useful in the treatment of diseases caused by bacteria which are resistant to other antibiotics such as penicillin compounds, and in the treatment of penicillin-sensitive patients. In many instances it is desirable to employ a cephalosporin antibiotic which exhibits activity against both Gram-positive and Gram-negative microorganisms, and a significant amount of research has been directed to the development of various types of broad spectrum cephalosporin antibiotics.

Thus, for example, in our British Patent Specification No. 1399086, we describe a novel class of cephalosporin antibiotics containing a 7β-(α-etherified oxyimino)acylamido side chain, the oxyimino group having the syn configuration. This class of antibiotic compounds is characterised by high antibacterial activity against a range of Gram-positive and Gram-negative organisms coupled with particularly high stability to β-lactamases produced by various Gram-negative organisms.

The discovery of this class of compounds has stimulated further research in the same area in attempts to find compounds which have improved properties, for example against particular classes of organisms, especially Gram-negative organisms. This interest is reflected in the very large numbers of patent applications which have been filed relating to cephalosporin antibiotics having particular substituents both on the 7β-acylamido side chain and at the 3-position of the cephalosporin nucleus.

For example, British Patent Specification No. 1576625 contains a generic definition of cephalosporin antibiotics having a 7β-(α-etherified oxyimino)acetamido side chain wherein the etherifying group is an aliphatic hydrocarbon group which may have suitable substituents (including, amongst a large number of possibilities a carboxy or protected carboxy group and a phenyl group substituted by up to three hydroxy groups), which side chain is further α-substituted by a group which inter alia may be an aminothiazolyl group. However none of the compounds specifically exemplified contains a carboxyphenylalkyl etherifying group. The 3-position group may also be selected from a large number of alternatives but vinyl and methyl groups are not included in the list substituents.

In British Patent Specification No. 1604971 a wide variety of cephalosporin antibiotics are generically disclosed in which the 7β-position side chain may be selected from inter alia a 2-(2-aminothiazol-4-yl)-2-(etherified oxyimino)acetamido group, in which the etherifying group, amongst very many possible meanings, may be an alkyl group (e.g. methyl) substituted by both phenyl and carboxy, although there is no specific exemplification of compounds having a carboxyphenylalkyl group and the preferred etherifying group is stated to be an unsubstituted methyl group. The 3-position group may also be selected from a large number of alternatives and possible 3-substituents within the generic definition include a methyl group. There is no disclosure of a vinyl group as a possible 3-substituent.

UK Patent Specification No. 2104888 generically discloses cephalosporin antibiotics in which the 7β-position side chain is a 2-(2-aminothiazol-4-yl)-2-(etherified oxyimino)acetamido group. The oxyimino etherifying group may inter alia be a carboxyphenylmethyl group in which the phenyl group may be substituted by a hydroxy group. The 3-position group is an isothiazolylthiomethyl or iminoalkylidenedithietane group.

In UK Patent Specification No. 2017702 the oxyimino etherifying group, according to the generic definition, may inter alia be an α-carboxyphenylmethyl radical. The 3-position substituent may be selected from a large number of alternatives including an alkyl group but there is no mention of a vinyl group as a possible 3-position substituent. In the compounds specifically exemplified, the carboxyphenylmethyl group is always combined with an acetoxymethyl group at the 3-position.

European Patent Specification No. 197409 generically discloses cephalosporin antibiotics in which the 7β-position side chain is a 2-(2-aminothiazol-4-yl)-2-(etherified oxyimino)acetamido group. The oxyimino etherifying group may be inter alia a catechol carboxymethyloxyimino group. The 3-position substituent may be selected from a number of alternatives but these do not include a vinyl group or a methyl group.

We have now discovered that by the selection of a (Z)-2-(2-aminothiazol-4-yl)-2-(etherified oxyimino)acetamido group at the 7β-position in combination with a vinyl or methyl group at the 3-position, and by the selection of a α-carboxy substituted phenylmethoxyimino group as the etherified oxyimino grouping, cephalosporin compounds having a particularly advantageous profile of activity (described in more detail below) against a wide range of commonly encountered pathogenic organisms and/or that are of use as intermediates in the preparation of other active compounds, may be obtained.

Accordingly, we provide cephalosporin compounds of the general formula (I)

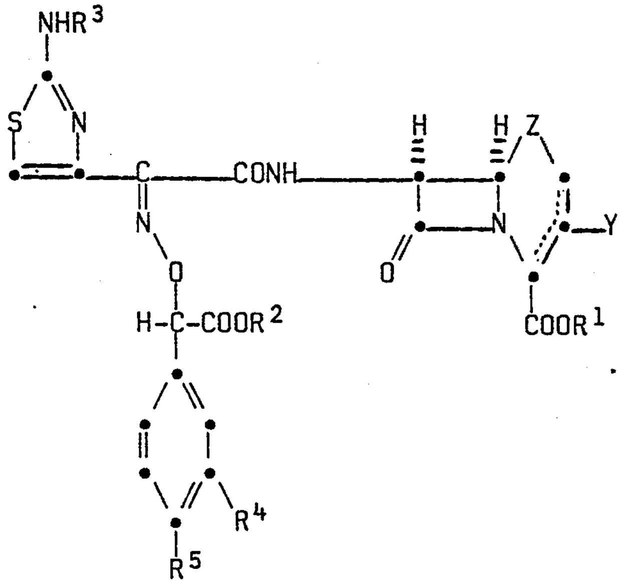

(I)

wherein

$R^1$ and $R^2$, which may be the same or different, each represents a hydrogen atom or a carboxyl blocking group;

$R^3$ represents a hydrogen atom or an amino protecting group;

$R^4$ and $R^5$ each represents a hydroxy or substituted hydroxy group or $R^4$ and $R^5$ together represent a cyclic protected diol group;

Y represents a methyl group or a vinyl group;

Z is $>S$ or $>S\rightarrow O$ ($\alpha$- or $\beta$-); the dotted line bridging the 2-, 3-, and 4-positions indicates that the compound is a ceph-2-em or ceph-3-em compound; and non-toxic salts and solvates (especially hydrates) thereof.

In the compounds of formula (I), where $R^1$ and/or $R^2$ represent carboxyl blocking groups, the blocking group may be for example the residue of an ester-forming aliphatic or araliphatic alcohol or of an ester-forming phenol, silanol or stannanol (the said alcohol, phenol, silanol or stannanol preferably containing from 1 to 20 carbon atoms) or a symmetrical or mixed anhydride blocking group derived from an appropriate acid. Particular examples of $R^1$ or $R^2$ include t-butyl, diphenylmethyl and p-nitrobenzyl.

Where $R^3$ is an amino protecting group, the protecting group may be for example a $C_{7-20}$ aralkyl group (for example a triphenylmethyl or 4-methoxybenzyl group), an acyl group, such as an optionally substituted $C_{1-6}$ alkanoyl group (for example a formyl or chloroacetyl group) or an optionally substituted $C_{1-6}$ alkoxycarbonyl group (for example a t-butoxycarbonyl or 2,2,2-trichloroethoxycarbonyl group), or a $C_{7-10}$ aralkyloxycarbonyl group (for example a benzyloxycarbonyl group) or a silyl group (for example a tri-methylsilyl group).

In general, compounds of formula (I) in which $R^3$ is a hydrogen atom are preferred.

When $R^4$ or $R^5$ represents a substituted hydroxy group it may be for example an acyloxy group [e.g. a formyloxy group or a group of formula $-OCOR^6$ (where $R^6$ is a $C_{1-8}$ alkyl group) for example an acetoxy group)], a carbonate group [for example a group of formula $-OCO_2R^6$ (where $R^6$ is as defined above)], a silyloxy group (for example a ($C_{1-4}$alkyl)silyloxy group such as a trimethylsilyloxy or a t-butyldimethylsilyloxy group) or a borate [$-OB(OR^7)_2$] or phosphate [$-OP(O)(OR^7)_2$] group (where $R^7$ represents $C_{1-4}$ alkyl).

Where $R^4$ and $R^5$ together form a cyclic protected diol grouping, this may be an alkylidenedioxy group, preferably having 1-20 carbon atoms, e.g. a methylenedioxy, ethylenedioxy or isopropylidenedioxy group which may carry one or more substituents e.g. phenyl, $C_{1-4}$ alkoxy, or oxo substituents, for example methoxymethylenedioxy, diphenylmethylenedioxy or carbonyldioxy groups; a cyclic borate group, for example $-OB(OH)O-$, a cyclic phosphate group, for example $-OP(O)(OH)O-$, or $-OP(O)(OR^7)O-$ (where $R^7$ is as defined above) and a cyclic silyl ether group, e.g. a di($C_{1-4}$alkyl)silyldioxy group for example a dimethylsilyldioxy group.

In general, such silyloxy, borate or phosphate groups represent protected hydroxy groups which may be cleaved to provide a compound of formula (I) having free hydroxyl groups.

In general, $R^4$ and $R^5$ is each preferably an acetoxy group or in particular a hydroxy group.

In the compounds of formula (I), Z is preferably $>S$.

Ceph-3-em compounds of the invention are particularly preferred.

Where the compound is to be used in medicine any ester of the carboxyl groups in the molecule should be a non-toxic metabolically labile ester function. Examples of non-toxic metabolically labile ester derivatives include acyloxyalkyl esters, for example, lower alkanoyloxy-methyl or -ethyl esters such as acetoxy-methyl or -ethyl or pivaloyloxymethyl esters, and alkoxycarbonyloxyethyl esters, for example, lower alkoxy-carbonyloxyethyl esters such as the ethoxycarbonyloxyethyl ester.

In addition to the above ester derivatives, the present invention includes within its scope the active compounds of the invention in the form of other physiologically acceptable equivalents, i.e. physiologically acceptable compounds which like the metabolically labile esters are converted in vivo into the parent antibiotic compounds of the invention.

Non-toxic salt derivatives which may be formed by reaction of the carboxyl group present in the compounds of formula (I) include inorganic base salts such as alkali metal salts (e.g. sodium and potassium salts) and alkaline earth metal salts (e.g. calcium salts); amino acid salts (e.g. lysine and arginine salts); organic base salts (e.g. procaine, phenylethylbenzylamine, dibenzylethylenediamine, ethanolamine, diethanolamine and N-methylglucosamine salts). Other non-toxic salt derivatives include acid addition salts, e.g. formed with hydrochloric, hydrobromic, sulphuric, nitric, phosphoric, formic and trifluoroacetic acids. The salts may also be in the form of resinates formed with, for example, a polystyrene resin or crosslinked polystyrene divinylbenzene copolymer resin containing amino or quaternary amino groups or sulphonic acid groups, or with a resin containing carboxyl groups, e.g. a polyacrylic acid resin. Soluble base salts (e.g. alkali metal salts such as the soldium salt) of the compounds of formula (I) may be used in therapeutic applications because of the rapid distribution of such salts in the body upon administration. Where, however, insoluble salts of compounds (I) are desired in a particular application, e.g. for use in depot preparations, such salts may be formed in conventional manner, for example with appropriate organic amines.

The compounds according to the invention are syn isomers. The syn isomeric form is defined by the configuration of the

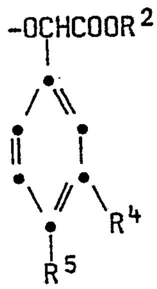

group with respect to the carboxamido group. In this specification, the syn configuration is denoted structurally as:

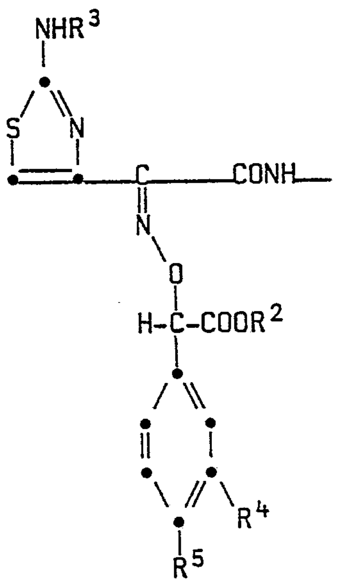

It will be understood that since the compounds according to the invention are geometric isomers, some admixture with the corresponding anti isomer may occur.

It will further be appreciated that in the oxime etherifying group, the carbon atom adjacent to the oxy group is chiral and may therefore exist in either the R or S configuration. The invention thus includes within its scope all individual enantiomeric forms of the compounds of formula (I) as well as mixtures (including racemic mixtures) thereof. In general, compounds of formula (I) in which this chiral carbon atom has the S-configuration and R/S mixtures in which the S-isomer predominates are preferred.

The compounds according to the present invention may exist in tautomeric forms (for example in respect of

the 2-aminothiazolyl group) and it will be understood that such tautomeric forms, e.g. the 2-iminothiazolinyl form, are included within the scope of the invention.

As indicated previously, the compounds of the invention are active against a wide range of commonly encountered pathogenic organisms and/or of use as intermediates for the preparation of other active compounds. In general, when the compounds of the invention are to be used as intermediates the groups $R^1$ and $R^2$ will often be carboxyl blocking groups; the group $R^3$ will be an amino protecting group, and the groups $R^4$ and $R^5$ will often be protected hydroxy groups such as silyloxy, borate or phosphate groups, or together will be a cyclic protected diol group. Non-toxic derivatives wherein $R^4$ and/or $R^5$ represent acyloxy groups such as acetoxy groups may serve as either intermediates or as active compounds.

In general active compounds of the invention will be ceph-3-em compounds of formula (I) in which $R^1$, $R^2$ and $R^3$ each represent hydrogen atoms, Z represents >S and $R^4$ and $R^5$ which may be the same or different represent hydroxy or $C_{1-4}$ acyloxy groups for example acetoxy groups.

Important active compounds according to the invention have the formula (Ia)

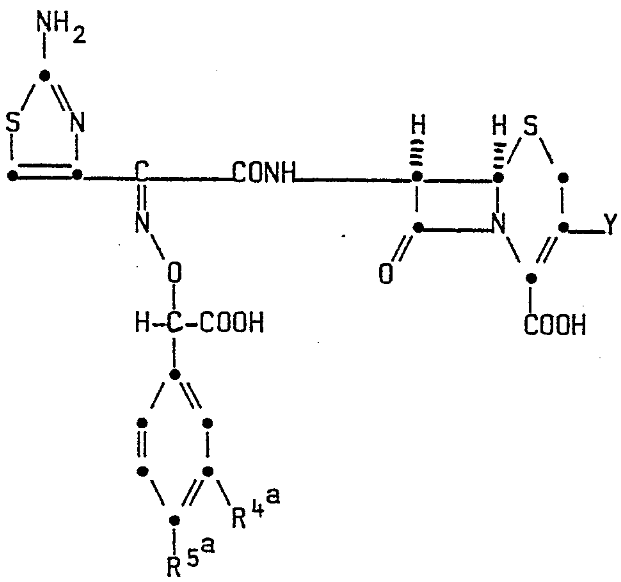

(Ia) .

wherein Y is as defined above;
$R^4{}_a$ is a hydroxy or acetoxy group;
$R^5{}_a$ is a hydroxy or acetoxy group;
and the non-toxic salts, solvates and metabolically labile esters thereof.

Preferred compounds according to the invention are:
(6R,7R,2'Z,S)-7-[2-Aminothiazol-4-yl)-2-[(carboxy)(3,4-dihydroxyphenyl)methoxyimino]acetamido]-3-methylceph-3-em-4-carboxylic acid;
(6R,7R,2'Z,S)-7-[2-Aminothiazol-4-yl)-2-[(carboxy)(3,4-dihydroxyphenyl)methoxyimino]acetamido]-3-vinylceph-3-em-4-carboxylic acid; and non toxic salts, solvates and metabolically labile esters thereof.

Compounds according to the invention exhibit broad spectrum antibiotic activity both in vitro and in vivo. They have high activity against both Gram-positive and Gram-negative organisms, including many β-lactamase producing strains. The compounds also possess high stability to β-lactamases produced by a range of Gram-negative and Gram-positive organisms.

Compounds acccording to the invention have been found to exhibit high activity against strains (including penicillinase-producing strains) of Gram-positive bacteria such as Staphylococcus aureus, Staphylococcus epidermidis and Streptococcus species. This is coupled with excellent activity against Pseudomonas species and also with high activity against various members of the Enterobacteriaceae (e.g. strains of Escherichia coli, Klebsiella pneumoniae, Enterobacter cloacae, Serratia marcescens, Proteus mirabilis and indole-positive Proteus organisms such as Proteus vulgaris, and Proteus morganii species), and strains of Haemophilus influenzae and Acinetobacter calcoaceticus. This combination of high activity against Gram-positive organisms with high activity against Gram-negative organisms, more particularly against Pseudomonas, that is possessed by the compounds of the invention is unusual and particularly advantageous.

Compounds of the invention may be used for treating a variety of diseases caused by pathogenic bacteria in human beings and animals, such as respiratory tract infections and urinary tract infections.

The antibiotic compounds of the invention may be formulated for administration in any convenient way, by analogy with other antibiotics and the invention therefore includes within its scope pharmaceutical compositions comprising an antibiotic compound in accordance with the invention adapted for use in human or veterinary medicine. Such compositions may be presented for use in conventional manner with the aid of any necessary pharmaceutical carriers or excipients.

The antibiotic compounds according to the invention may be formulated for injection and may be presented in unit dose form, in ampoules, or in multi-dose containers, if necessary with an added preservative. The compositions may also take such forms as suspensions, solutions, or emulsions in oily or aqueous vehicles, and may contain formulatory agents such as suspending, stabilising and/or dispersing agents. Alternatively the active ingredient may be in powder form for reconstitution with a suitable vehicle, e.g. sterile, pyrogen-free water, before use.

If desired, such powder formulations may contain an appropriate non-toxic base in order to improve the water-solubility of the active ingredient and/or to ensure that when the powder is reconstituted with water, the pH of the resulting aqueous formulation is physiologically acceptable. Alternatively the base may be present in the water with which the powder is reconstituted. The base may be, for example, an inorganic base such as sodium carbonate, sodium bicarbonate or sodium acetate, or an organic base such as lysine or lysine acetate.

The composition may also be presented in a form suitable for absorption by the gastro-intestinal tract, for example, tablets, capsules, syrups or suspensions for oral administration, and suppositories.

Compositions for veterinary medicine may, for example, be formulated as intramammary preparations in either long acting or quick-release bases.

The compositions may contain from 0.1% upwards, e.g. 0.1-99% of the active material, depending on the method of administration. When the compositions comprise dosage units, each unit will preferably contain 100-3000 mg of the active ingredient e.g. 200-2000 mg. The daily dosage for adult human treatment will preferably range from 200 to 12000 mg e.g. 1000-9000 mg per day, depending inter alia on the nature of the infection and the route and frequency of administration. In general, intravenous or intramuscular administration will be employed, for example using 400 to 4000 mg per day of the active ingredient in adult human treatment. It will be appreciated that in some circumstances, for example, in the treatment of neonates, smaller dosage units and daily dosages may be desirable.

The antibiotic compounds according to the invention may be administered in combination with other therapeutic agents such as antibiotics, for example penicillins or other cephalosporins.

The compounds of the invention may be prepared by a number of processes, discussed below.

Thus, according to another embodiment of the invention we provide a process for the preparation of an antibiotic compound of general formula (I) as hereinbefore defined or a non-toxic salt thereof by acylating a compound of formula (II)

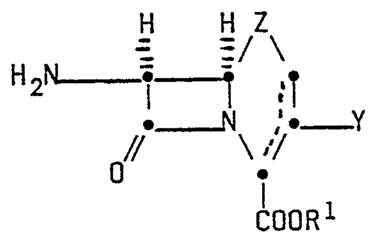

(II)

(wherein $R^1$, Y, Z and the dotted line are as defined above) or a salt, e.g. an acid addition salt (formed with, for example, a mineral acid such as hydrochloric, hydrobromic, sulphuric, nitric or phosphoric acid or an organic acid such as methanesulphonic or toluene-p-sulphonic acid) or a 7-N-silyl derivative thereof, with an acid of formula (III)

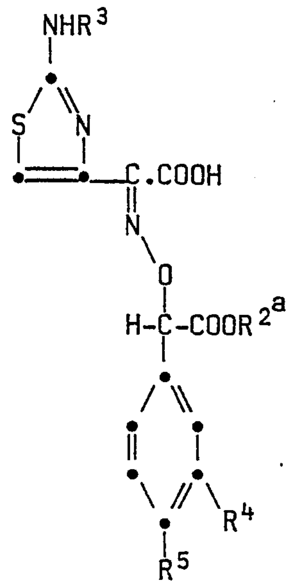

(III)

(wherein $R^3$, $R^4$ and $R^5$ are as defined above and $R^2{}_a$ represents a carboxyl blocking group) or a salt thereof, or with an acylating agent corresponding thereto;
whereafter, if necessary and/or desired in each instance, any of the following reactions, in any appropriate sequence, may be carried out:-

i) conversion of a $\Delta^2$-isomer into a desired $\Delta^3$-isomer,
ii) reduction of a compound wherein Z is $>S\rightarrow O$ to form a compound wherein Z is $>S$,
iii) conversion of a carboxyl group into a non-toxic metabolically labile ester function,
iv) formation of a non-toxic salt,
v) removal of any carboxyl blocking and/or N-protecting groups, and
vi) removal of any hydroxy blocking groups.

The above reactions i) to vi) may be carried out in conventional manner.

In the above described acylation process, the starting material of formula (II) is preferably a compound wherein Z is $>S$ and the dotted line represents a ceph-3-em compound.

Acylating agents which may be employed in the preparation of compounds of formula (I) include acid halides, particularly acid chlorides or bromides. Such acylating agents may be prepared by reacting an acid (III) or a salt thereof with a halogenating agent e.g. phosphorus oxychloride, thionyl chloride or oxalyl chloride.

Acylations employing acid halides may be effected in aqueous and non-aqueous reaction media, conveniently at temperatures of from -50 to +50°C, preferably -40 to +30°C, if desired in the presence of an acid binding agent. Suitable reaction media include aqueous ketones such as aqueous acetone, aqueous alcohols such as aqueous ethanol, esters such as ethyl acetate, halogenated hydrocarbons such as methylene chloride, amides such as dimethylacetamide, nitriles such as acetonitrile, or mixtures of two or more such solvents. Suitable acid binding agents include tertiary amines (e.g. triethylamine or dimethylaniline), inorganic bases (e.g. calcium carbonate or sodium bicarbonate), and oxiranes such as lower 1,2-alkylene oxides (e.g. ethylene oxide or propylene oxide) which bind hydrogen halide liberated in the acylation reaction.

Acids of formula (III) may themselves be used as acylating agents in the preparation of compounds of formula (I). Acylations employing acids (III) are desirably conducted in the presence of a condensing agent, for example a carbodiimide such as N,N'-dicylco hexylcarbodiimide or N-ethyl-N'-γ-dimethylaminopropylcarbodiimide; a carbonyl compound such as carbonyldiimidazole; or an isoxazolium salt such as N-ethyl-5-phenylisoxazolium perchlorate; or N-ethoxycarbonyl-2-ethoxy-1,2-dihydroquinoline.

Acylation may also be effected with other amide-forming derivatives of acids of formula (III) such as, for example, an activated ester, a symmetrical anhydride or a mixed anhydride (e.g formed with pivalic acid or with a haloformate, such as a lower alkyl-haloformate). Mixed anhydrides may also be formed with phosphorus acids (for example phosphoric or phosphorus acids), sulphuric acid or aliphatic or aromatic sulphonic acids (for example toluene-p-sulphonic acid). An activated ester may conveniently be formed in situ using, for example, 1-hydroxybenzotriazole in the presence of a condensing agent as set out above. Alternatively, the activated ester may be preformed.

Acylation reactions involving the free acids or their above-mentioned amide-forming derivatives are desirably effected in an anhydrous reaction medium, e.g. methylene chloride, tetrahydrofuran, dimethylformamide, acetonitrile, dimethylacetamide or dimethyl sulphoxide.

An alternative method of activation is, for example, by reacting an acid of formula (III) with a solution or suspension preformed by adding a carbonyl halide, in particular oxalyl chloride or phosgene, or a phosphoryl halide such as phosphorus oxychloride to a solvent such as a halogenated hydrocarbon, for example

methylene chloride, containing a lower acyl tertiary amide such as N,N-dimethylformamide. The activated form of the acid of formula (III) may then be reacted with a 7-amino compound of formula (II) in a suitable solvent of mixture of solvents for example halogenated hydrocarbons e.g. dichloromethane; alcohols such as an alkanol, e.g. ethanol or industrial methylated spirits; esters, e.g. ethyl acetate; ethers, e.g. tetrahydrofuran or dioxan; ketones, e.g. acetone; amides, e.g. dimethylacetamide; acetonitrile; water and mixtures thereof. The acylation reaction may conveniently be effected at temperatures of from -50° to 50°C, preferably -40° to +30°C, if desired in the presence of an acid binding agent, for example as described above (e.g. dimethylaniline, triethylamine or sodium bicarbonate).

If desired, the above acylation reactions may be carried out in the presence of a catalyst such as 4-dimethylaminopyridine.

The acids of formula (III) and acylating agents corresponding thereto may, if desired and where appropriate, be prepared and employed in the form of their acid addition salts. Thus, for example, acid chlorides may conveniently be employed as their hydrochloride salts, and acid bromides as their hydrobromide salts.

For use as starting materials for the preparation of compounds of general formula (I) according to the invention, compounds of general formula (III) and the amide forming derivatives thereof such as acid halides and anhydrides corresponding thereto in their syn isomeric form or in the form of mixtures of the syn isomers and the corresponding anti isomers containing at least 90% of the syn isomer are preferably used.

Acids of formula (III) and their derivatives may be prepared by etherification of a compound of formula (IV)

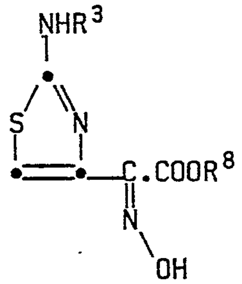

(IV)

(wherein $R^3$ is as hereinbefore defined and $R^8$ represents a hydrogen atom or a carboxyl blocking group) or a salt thereof, by selective reaction with a compound of general formula (V)

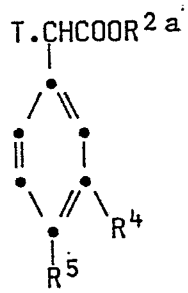

(V)

(wherein $R^2{}_a$, $R^4$ and $R^5$ are as hereinbefore defined and T is chloro, bromo or iodo atom, a sulphate group or a sulphonate group, such as tosylate), followed by removal of any carboxyl blocking group $R^8$. Separation of isomers may be effected either before or after such etherification.

The etherification reaction is conveniently carried out in the presence of a base, e.g. potassium carbonate or sodium hydride, and is preferably conducted in an organic solvent, for example dimethylsulphoxide, a cyclic ether such as tetrahydrofuran or dioxan, or an N,N-disubstituted amide such as dimethylformamide. Under these conditions the configuration of the oxyimino group is substantially unchanged by the etherification reaction. When the compound of formula (IV) is employed in the form of a free acid or a salt with a base, the etherification reaction is generally carried out in the presence of a strong base, e.g. potassium t-butoxide, sufficient base being added to form a dianion. Furthermore, the reaction should be effected in the presence of a base if an acid addition salt of a compound of formula (IV) is used, the amount of base being sufficient to neutralise rapidly the acid in question.

Acids of formula (III) may also be prepared by reaction of a compound of formula (VI)

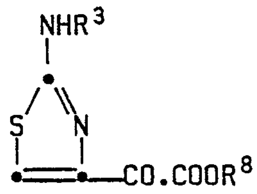

(VI)

(wherein $R^3$ and $R^8$ are as hereinbefore defined) with a compound of formula (VII)

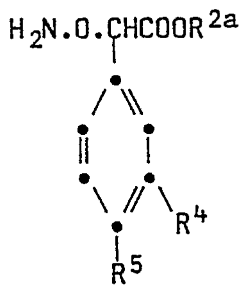

(VII)

(wherein $R^2{}_a$, $R^4$ and $R^5$ are as hereinbefore defined) followed by removal of any carboxyl blocking group $R^8$, and where necessary the separation of syn and anti isomers.

The reaction is conveniently carried out in a solvent such as dimethylformamide, dimethylacetamide, dimethyl sulphoxide, tetrahydrofuran or methanol, all optionally in the presence of water, at a temperature of -20° to +50°C, preferably 0° to 30°C.

The acids of formula (III) may be converted into the corresponding acid halides and anhydrides and acid addition salts by conventional methods, for example as described hereinabove.

Intermediates of formula (VII) may be prepared by treating compounds of formula (VIII)

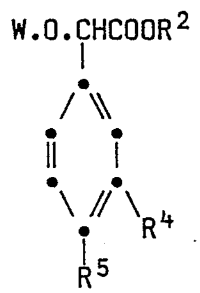

(VIII)

(wherein W is an imido group, for example a phthalimido, succinimido or maleimido group and $R^2$, $R^4$ and $R^5$ are as defined above) with a hydrazine reagent such as hydrazine hydrate or an alkyl hydrazine such as methyl hydrazine. The reaction will generally be performed in an inert solvent, for example dioxan or a halogenated hydrocarbon such as methylene chloride at a low temperature, for example -70° to +30°C. A carboxyl group $R^2$ may be protected by conventional methods.

Intermediates of formula (VIII) may be prepared by alkylation with compounds of formula (IX)

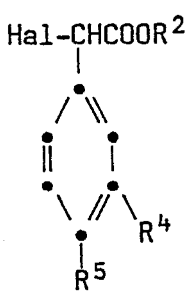

(IX)

[wherein Hal is a halogen atom such as a chlorine or bromine atom and $R^4$ and $R^5$ are as defined above) of an appropriate N-hydroxyimide, (e.g. N-hydroxyphthalimide, N-hydroxysuccinimide or N-hydroxymaleimide] in the presence of a base such as triethylamine in a solvent such as acetonitrile at for example -10° to +30°C.

Intermediates of formula (IX) are either known compounds or may be prepared using methods analogous to those used for the preparation of the known compounds.

Compounds of formula (II) used as starting materials in the acylation process may generally be prepared by N-deacylating compounds of formula (X)

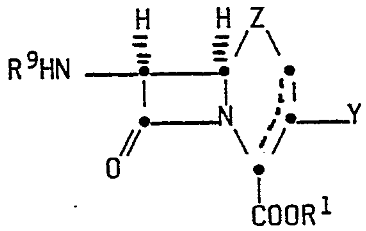

(X)

(wherein $R^1$, Y,Z and the dotted line are as defined for formula (I) and $R^9$ represents an acyl group such as a carboxylic acyl group, for example formyl, acetyl, optionally substituted phenylacetyl, optionally substituted phenoxyacetyl, thienylacetyl or 5-aminoadipoyl, or the latter having one or both of the carboxyl and amino groups thereof blocked; or an alkoxycarbonyl group, for example t-butoxycarbonyl; or the group $R^9$ and the adjacent hydrogen atom together represent a diacyl grouping derived from a carboxylic acid, such as a phthalimido or maleimido group, $R^9NH$ thus being an N-attached heterocyclic group). The N-deacylation may be effected in conventional manner, for example using phosphorus pentachloride as described in British Patent Specification No. 1241655.

Starting materials of formula (II) in which Y is a vinyl group are described in our British Patent Specification No. 1342241.

The reaction product from the above process may be separated from the reaction mixture, which may contain, for example, unchanged cephalosporin starting material and other substances, by a variety of processes including recrystallisation, ionophoresis, column chromatography, high pressure liquid chromatography, ion-exchange chromatography or chromatography on macroreticular resins.

A $\Delta^2$-cephalosporin ester derivative obtained in accordance with the processes of the invention may be converted into the corresponding desired $\Delta^3$-derivative by, for example, treatment of the $\Delta^2$-ester with a base, such as pyridine or triethylamine.

A ceph-2-em reaction product may alos be oxidized to yield the corresponding ceph-3-em 1-oxide, for example by reaction with a peracid, e.g. peracetic or m-chloroperbenzoic acid; the resulting sulphoxide may subsequently be reduced as described hereinafter to yield the corresponding desired ceph-3-em sulphide.

Where a compound is obtained in which Z is $>S{\rightarrow}0$ this may be converted into the corresponding sulphide by, for example, reduction of the corresponding acyloxysulphonium or alkoxysulphonium salt prepared in situ by reaction with e.g. acetyl chloride in the case of an acetoxysulphonium salt, reduction being effected by, for example, sodium dithionite or by iodide ion as in a solution of potassium iodide in a solvent e.g. acetic acid, acetone, tetrahydrofuran, dioxan, dimethylformamide or dimethylacetamide. The reaction may be effected at a temperature of from -20° to +50°C.

In the oxidation and reduction processes described above, the groups $R^4$ and $R^5$ in the starting materials are desirably other than hydroxyl groups.

Metabolically labile ester derivatives of the carboxyl groups in the compounds of formula (I) may be prepared by reacting a compound of formula (I) or a salt or protected derivative thereof with the appropriate esterifying agent such as an acyloxyalkyl halide or alkoxycarbonyloxyalkyl halide (e.g. iodide) conveniently in an inert organic solvent such as dimethylformamide or acetone, followed, where necessary, by removal of any protecting groups.

Base salts of the compounds of formula (I) may be formed by reacting an acid of formula (I) with an appropriate base. Thus, for example, sodium or potassium salts may be prepared using the respective acetate, 2-ethylhexanoate or hydrogen carbonate salt. Acid addition salts may be prepared by reacting a compound of formula (I) or a metabolically labile ester derivative thereof with the appropriate acid.

Where a compound of formula (I) is obtained as a mixture of isomers, the syn isomer may be obtained by, for example, conventional methods such as crystallisation or chromatography.

It should be appreciated that in some of the above transformations it may be necessary to protect any sensitive groups in the molecule of the compound in question to avoid undesirable side reactions. Examples of suitable protecting groups are given in "Protective Groups in Organic Synthesis" by Theodora W. Greene (John Wiley and Sons, 1981).

For example, during any of the reaction sequences referred to above it may be necessary to protect the $NH_2$ group of the aminothiazolyl moiety, for example by tritylation, acylation (e.g. chloroacetylation or formylation), protonation or other conventional method. The protecting group may thereafter be removed in any convenient way which does not cause breakdown of the desired compound, e.g. in the case of a trityl group by using an optionally halogenated carboxylic acid, e.g. acetic acid, formic acid, chloroacetic acid or trifluoroacetic acid or using a mineral acid, e.g. hydrochloric acid or mixtures of such acids, preferably in the presence of a protic solvent such as water, or, in the case of a chloroacetyl group, by treatment with thiourea.

Similarly, the hydroxy groups of the catechol moiety may need to be protected during any of the above reaction sequences. Hydroxy protecting groups which may be removed under mild conditions will generally be suitable, for example acetyl or silyl groups. Such groups may be introduced in conventional manner and, when desired, removed such that breakdown of the product does not occur. For example in the case of an acetyl group, the group may be removed by solvolysis with an aqeuous solvent such as aqueous methanol or aqueous ethanol in the presence of a base for example sodium bicarbonate, ammonium hydroxide, ammonium carbonate or ammonium carbamate. In the case of a silyl group, a triethylsilyl group may be cleaved for example by treatment with a dilute aqueous acid.

Carboxyl blocking groups used in the preparation of compounds of formula (I) or in the preparation of necessary starting materials are desirably groups which may readily be split off at a suitable stage in the reaction sequence, conveniently at the last stage. It may, however, be convenient in some instances to employ non-toxic metabolically labile carboxyl blocking groups such as acyloxy-methyl or -ethyl groups (e.g. acetoxy-methyl or -ethyl or pivaloyloxymethyl) and retain these in the final product to give an appropriate ester derivative of a compound of formula (I).

Suitable carboxyl blocking groups are well known in the art, a list of representative blocked carboxyl groups being included in British Patent No. 1399086. Preferred blocked carboxyl groups include aryl lower alkoxycarbonyl groups such as p-methoxybenzyloxycarbonyl, p-nitrobenzyloxycarbonyl and diphenylmethoxycarbonyl; lower alkoxycarbonyl groups such as t-butoxycarbonyl; and lower haloalkoxycarbonyl groups such as 2,2,2-trichloroethoxycarbonyl. The carboxyl blocking group may subsequently be removed by any of the appropriate methods disclosed in the literature; thus, for example, acid catalysed hydrolysis, reduction or enzymically-catalysed hydrolysis.

Where a particular enantiomer of a compound of formula (I) is required starting materials having the desired stereochemical configuration should be used in the above processes. Such intermediates may be obtained using conventional resolution processes. Thus for example enantiomeric intermediates of formula (VIII) wherein $R^2$ is -H in which the chiral carbon atom is in the (R) or (S) configuration may be obtained by reaction of a mixture of enantiomers with a resolving agent such as a chiral organic base [e.g. R-(+)-α-methylbenzylamine] in a solvent such as acetone or acetonitrile to form the corresponding diastereomeric salts. The salts may then be separated by known methods and the desired chiral intermediate of formula (VIII) regenerated by treatment with an aqeuous acid, e.g. aqueous hydrochoric acid, at for example room temperature.

The following Examples illustrate the invention. All temperatures are in °C. Sorbsil U30 is silica gel manufactured by Joseph Crosfield and Son of Warrington, Cheshire, England. Sorbsil and Nujol are trade marks.

Intermediate 1

2-(3,4-Dioxycarbonylphenyl)-2-(phthalimidooxy)acetic acid

A solution of N-hydroxyphthalimide (14.6g) and triethylamine (25ml) in acetonitrile (50ml) was added to a stirred suspension of 2-(3,4-dioxycarbonylphenyl)-2-chloroacetic acid (20.5g) in acetonitrile (100ml) at -2° over 20 minutes. After a further hour at about 0°, a solution of concentrated hydrochloric acid (7.5ml) in water (100ml) was added rapidly. Water (100ml) was added slowly and the initial oil liberated crystallised, aided by seeding. The mixture was filtered and the filter-cake was washed with water, and ethyl acetate:petroleum ether (bp 40-60°) (1:2) and dried to give the title compound (30g), m.p. 183 to 185°;
δ($d_6$ DMSO) 5.84 (s; OCH), 7.53 (s; catechol 5-H and 6-H), 7.69 (s; catechol 2-H), and 7.85 (s; phthamido protons).

Intermediate 2

(a) (R)-(+)-α-Methylbenzylamine salt of (S)-2-(3,4-Dioxycarbonylphenyl)-2-(phthalmidooxy)acetic acid

A solution of R-(+)-α-methylbenzylamine (16.3ml) in acetone (100ml) was added rapidly to a magnetically stirred solution of Intermediate 1 (45g) in acetone (1.25 litres) at 21° under nitrogen. After 30 minutes the mixture was filtered and the precipitate was washed thoroughly with acetone to yield the title compound (16.57g), $[\alpha]_D^{21}$ + 242° (c 1.07, EtOH);
δ ($d_6$ DMSO) 1.46 (d, J7Hz, $CH_3CH$), 5.48 (s; OCH), 7.3 to 7.6 (m; aromatic protons) and 7.80 (s; phthalimido protons).

(b) Diphenylmethyl (S)-2-(3,4-Dioxycarbonylphenyl)-2-(phthalimidooxy) acetate

2M Hydrochloric acid (10ml) was added to a stirred suspension of Intermediate 2(a) (5.0g) in water (30ml) under nitrogen at 21°. After 2 minutes, a solution of diphenyldiazomethane containing one equivalent in methylene chloride (11ml) was added. After stirring vigorously for 35 minutes, the organic layer was separated and added dropwise to stirred ethanol (150ml). The mixture was stirred at 21° for 10 minutes and then stored at 4° for 1 hour. The crystals were collected by filtration, washed with ethanol and dried to give the title compound (3.59g), m.p. 135 to 135.5°;
$[\alpha]_D^{21}$ + 112.6° (c 1.18, ethyl acetate).

Intermediate 3

(S)-(3,4-Dihydroxyphenyl)(diphenylmethoxycarbonyl)methoxyamine

Intermediate 2(b) (5.71g) was stirred in methanol (390ml) with 1M hydrochloric acid (5.5ml) at ca 40° for 4.5 hours. The solution was concentrated and mixed with methylene chloride. After washing twice with water, the solution was dried over magnesium sulphate and evaporated to a foam. A solution of this foam in methylene chloride (176ml) was cooled to -50° with stirring under nitrogen and hydrazine hydrate (1.5ml) added. The mixture was allowed to warm slowly to 21° and stirred. After 5.75 hours the mixture was filtered and the filter-cake was leached with methylene chloride. The combined filtrates were diluted with ethyl acetate and washed with citric acid solution and brine. After drying over magnesium sulphate, evaporation gave the title compound (4.35g) as a foam:

$[\alpha]_D^{21}$ + 17.8° (c 1.03, methanol);

δ ($d_6$ DMSO) 5.02 (s; OCHCO), 6.68 and 6.81 (m, thiazole H, OCH and catechol protons), 7.1 to 7.4 (m, aromatic protons) 9.04 (m, OH).

Intermediate 4

(Z,S)-2-(2-Aminothiazol-4-yl)-2-[(3,4-dihydroxyphenyl)(diphenylmethoxycarbonyl)]methoxyiminoacetic acid

2-(2-Aminothiazol-4-yl)glyoxylic acid (1.91g) was added over 3 minutes to a solution of Intermediate 3 (4.20g) in N,N-dimethylformamide (22ml) at 3° with stirring. After a further 30 minutes with ice-water cooling, the solution was allowed to warm to 21° over 1.5 hours and then added dropwise to an ice-water mixture (110g) with stirring for 20 mintues. The precipitate was collected by filtration, washed with water and dried. It was resuspended in methylene chloride (45ml) and stirred for 15 minutes before filtration. The filter-cake was washed with methylene chloride and dried to give the title compound (3.76g);

$[\alpha]_D^{21}$ + 25.4° (c 1.02, methanol);

δ ($d_6$ DMSO) 5.59 (s; OCHCO), 6.6 to 6.9 (m, thiazole H, OCH, and catechol protons), 7.0 to 7.5 (m, $NH_2$ and phenyl protons) and 9.06 and 9.13 (2s, OH).

Example 1

Diphenylmethyl (6R,7R,2'Z,S)-7-[2-(2-aminothiazol-4-yl)-2-[(3,4-dihydroxyphenyl)(diphenylmethoxycarbonyl)methoxyimino]acetamido]-3-vinylceph-3-em-4-carboxylate

A solution of diphenylmethyl (6R,7R)-7-amino-3-vinylceph-3-em-4-carboxylate (1.688g), Intermediate 4 (2.21g), and 1-hydroxybenzotriazole hydrate (650mg), in dry tetrahydrofuran (40ml) was treated with a solution of dicyclohexylcarbodiimide (1.31g) in dry tetrahydrofuran (10ml). The mixture was stirred at 20° for 21 hours and dicyclohexylurea removed by filtration. The filtrate was evaporated in vacuo and the resulting foam taken up in ethyl acetate. The solution was stored at room temperature for 1 hour during which time more dicyclohexylurea crystallised out. This was removed by filtration and the filtrate evaporated to give a foam (5.25g). A portion (4.6g) of this material was chromatographed on silica (200g) using methylene dichloride:ethyl acetate:acetic acid (40:20:1) as eluent. Similar fractions were combined and evaporated in vacuo to give a foam (1.2g). A solution of this material, in ethyl acetate (10ml), was run into petroleum ether (bp 40-60, 1000ml) and the precipitated solid collected by filtration and dried in vacuo to give the title ester (1.026g) as an amorphous solid;

λ infl (ethanol) 230.4 nm ($E_{1cm}^{1\%}$ 359) and $\lambda_{max}$ 290.4 nm ($E_{1cm}^{1\%}$ 267);

δ ($d_6$ DMSO) includes, 9.66 (d,8Hz,1H), 9.5 (s,1H), 8.7 (s,1H), 5.83 (dd,8 and 5Hz,1H), 5.61 (d, 18Hz,1H), 5.6 (s,1H), 5.31 (d,11Hz,1H), 5.18 (d,5.18Hz,1H) and 3.61 and 3.47 (ABq, 18Hz,2H).

Example 2

(6R,7R,2'Z,S)-7-[2-(2-Aminothiazol-4-yl)-2-[3,4-dihydroxyphenyl)(carboxy)methoxyimino]acetamido]-3-vinylceph-3-em-4-carboxylic acid

The compound of Example 1 (850mg) was treated with anisole (0.85ml) and the resulting syrup was diluted with trifluoroacetic acid (3.4ml) at 20°. After 5 minutes the solution was run into diisopropyl ether (ca 200ml). The precipitated solid was collected by filtration, washed with diisopropyl ether, and dried in vacuo at 20° to give the title acid (561mg) as an amorphous solid;

λ max (pH6 buffer) 232.8 ($E_{1cm}^{1\%}$ 369), 286 ($E_{1cm}^{1\%}$ 369) and 286 nm ($E_{1cm}^{1\%}$ 398).

δ ($d_6$ DMSO) includes 9.52 (d,8Hz,1H), 5.78 (dd,8 and 5Hz, 1H) 5.6 (d,18Hz,1H), 5.38 (s,1H), 3.35 (d,11Hz,1H), 5.14 (d,5Hz,1H) and 3.68 and 3.5 (ABq,18Hz,2H).

Analysis showed that the product contained 0.5 mole of trifluoroacetic acid.

Example 3

Diphenylmethyl (6R,7R,2'Z,S)-7-[2-(2-aminothiazol-4-yl)-2-[(3,4-dihydroxyphenyl)(diphenylmethoxycarbonyl)methoxyimino]acetamido]-3-methylceph-3-em-4-carboxylate

Intermediate 4 (1.76g) was stirred with diphenylmethyl (6R,7R)-7-amino-3-methylceph-3-em-4-carboxylate (1.29g), 1-hydroxybenzotriazole hydrate (0.52g) and N,N'-dicyclohexylcarbodiimide (1.4g) in THF (40ml) at 21° for 22.5 hours. The mixture was filtered and the filter-cake was leached with ethyl acetate. The combined filtrates were evaporated and the residue in methylene chloride was loaded onto a column of Sorbsil U30 (150g) set up in 25% ethyl acetate-petroleum ether (bp 40-60°). Elution with increasing concentrations of ethyl acetate (25%, 50%, 75%, 100%) afforded a partially purified product. After removal of solvent the product was taken up in methylene chloride and chromatographed on Sorbsil U30 (150g) using initially chloroform as solvent and then increasing concentrations of chloroform-methanol (1%, 2%, 3%). Evaporation of the appropriate eluate gave the title compound (1.51g);

$[\alpha]_D^{21}$ +38.1° (c 1.35, DMSO);

$\lambda_{infl}$ (EtOH) includes 231.2 ($E_{1cm}^{1\%}$ 318), 272.4 ($E_{1cm}^{1\%}$ 140) and 298.8nm ($E_{1cm}^{1\%}$ 93).

Example 4

(6R,7R,2'Z,S)-7-[2-(2-Aminothiazol-4-yl)-2-[(carboxy)(3,4-dihydroxyphenyl)methoxyimino]acetamido]-3-methylceph-3-em-4-carboxylic acid, trifluoroacetic acid salt

The compound of Example 3 (1.38g) was dissolved in anisole (3ml) and trifluoroacetic acid (15ml) was added. After two minutes at 21°, diisopropyl ether (100ml) was added and the precipitate was collected by filtration, washed with diisopropyl ether and dried to give the title compound (710mg);

$[\alpha]_D^{21}$ +96.9° (c 1.01, DMSO);

$\lambda$max (pH6 buffer) 237.6nm ($E_{1cm}^{1\%}$ 385) and $\lambda$infl includes 296.8nm ($E_{1cm}^{1\%}$ 134).

**Claims**

1. Compounds of formula (I)

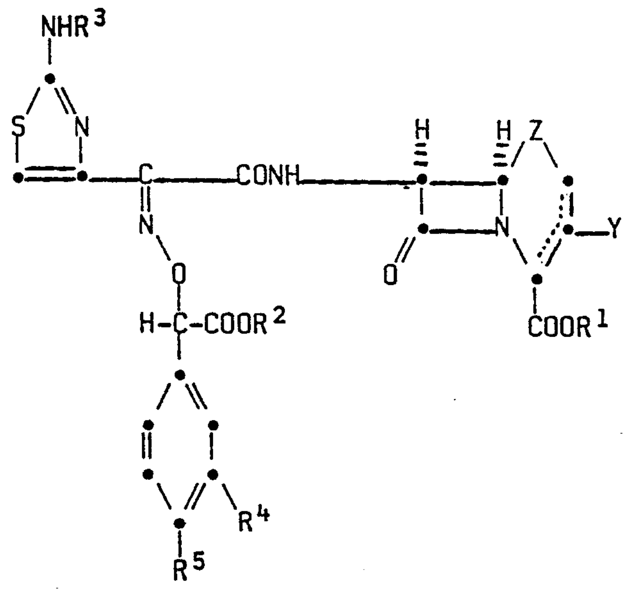

(I)

wherein

$R^1$ and $R^2$, which may be the same or different, each represents a hydrogen atom or a carboxyl blocking group;

$R^3$ represents a hydrogen atom or an amino protecting group;

$R^4$ and $R^5$ each represents a hydroxy or substituted hydroxy group or $R^4$ and $R^5$ together represent a cyclic protected diol group;

Y represents a methyl or vinyl group;

Z is -S- or -SO- (α- or β-) and the dotted line bridging the 2-, 3-, and 4-positions indicates that the compound is a ceph-2-em or ceph-3-em compound; and salts and solvates thereof.

2. Compounds as claimed in claim 1 of formula (Ia)

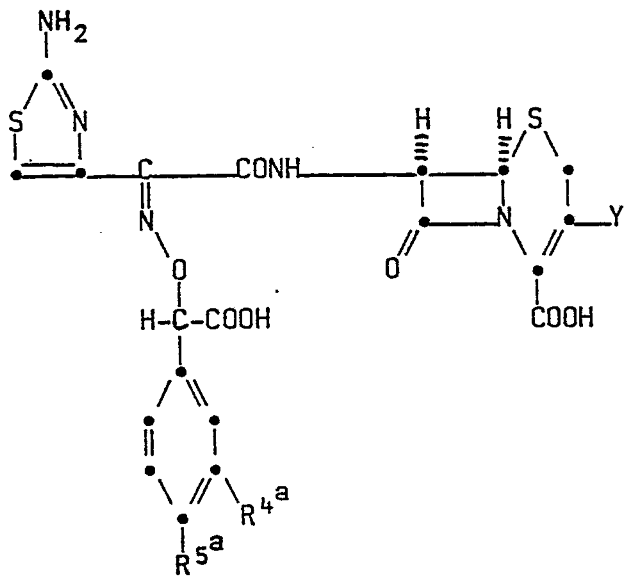

(Ia)

wherein Y is as defined in claim 1;

R4a is a hydroxy or acetoxy group;

R5a is a hydroxy or acetoxy group;

and the non-toxic salts and metabolically labile esters thereof.

3. Compounds of formula (Ia) as claimed in claim 2 in which the oxime etherifying group has the S-configuration.

4. A compound selected from (6R,7R,2'Z,S)-7-[2-(2-aminothiazol-4-yl)-2-[(carboxy) (3,4-dihydroxyphenyl)methoxyimino]acetamido]-3-methyl-ceph-3-em-4-carboxylic acid;
(6R,7R,2'Z,S)-7-[2-(2-aminothiazol-4-yl)-2-[(carboxy) (3,4-dihydroxyphenyl)methoxyimino]acetamido]-3-vinyl-ceph-3-em-4-carboxylic acid;
and the non-toxic salts and metabolically labile esters thereof.

5. A process for the preparation of compounds of formula (I) as defined in claim 1 or salts or solvates thereof by acylating a compound of formula (II)

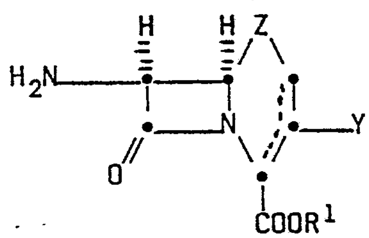

(II)

(wherein $R^1$, Y, Z and the dotted line are as defined in claim 1) or a salt or 7-N-silyl derivative thereof, with an acid of formula (III)

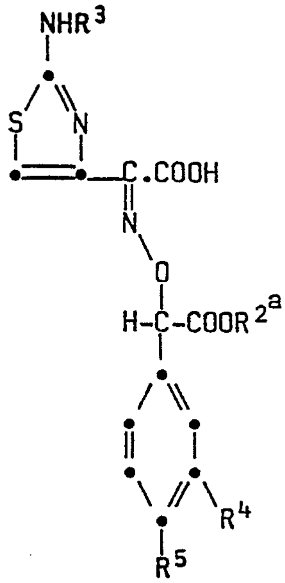

(III)

(wherein $R^3$, $R^4$ and $R^5$ are as defined in claim 1 and $R^{2a}$ is a carboxyl blocking group) or a salt thereof, or with an acylating agent corresponding thereto;
whereafter, if necessary and/or desired in each instance, any of the following reactions, in any appropriate sequence, are carried out:-

i) conversion of a $\Delta^2$-isomer into the desired $\Delta^3$-isomer,
ii) reduction of a compound wherein Z is -SO-to form a compound wherein Z is -S-,
iii) conversion of a carboxyl group into a non-toxic metabolically labile ester function,
iv) formation of a salt or solvate,
v) removal of any carboxy blocking and/or N-protecting groups, and
vi) removal of any hydroxy blocking groups.

6. A pharmaceutical composition comprising as active ingredient a compound of formula (Ia) as defined in claim 2.